(19) Europäisches Patentamt
European Patent Office
Office européen des brevets

(11) Veröffentlichungsnummer: **0 022 958**
**A1**

(12) # EUROPÄISCHE PATENTANMELDUNG

(21) Anmeldenummer: **80103765.6**

(22) Anmeldetag: **02.07.80**

(51) Int. Cl.[3]: **A 61 K 31/17**
C 07 C 127/19, C 07 C 157/09
C 07 C 149/437, C 07 C 147/00
C 07 C 143/58, C 07 C 143/72
C 07 D 521/00
//(C07D521/00, 317/46, 279/02, 251/22, 295/14, 215/42, 249/06, 213/75, 239/42, 215/38, 277/48, 279/08, 307/91, 277/82)

(30) Priorität: **14.07.79 DE 2928485**

(43) Veröffentlichungstag der Anmeldung:
**28.01.81 Patentblatt 81/4**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Kabbe, Hans-Joachim, Dr.**
**Walter-Flex-Strasse 16**
**D-5090 Leverkusen(DE)**

(72) Erfinder: **Klauke, Erich, Dr.**
**Eichendorffweg 8**
**D-5068 Odenthal(DE)**

(72) Erfinder: **Krause, Hans Peter, Dr.**
**Wilkhausstrasse 107**
**D-5600 Wuppertal 2(DE)**

(72) Erfinder: **Mardin, Mithat, Dr.**
**Theodor-Heuss-Strasse 50**
**D-5600 Wuppertal 1(DE)**

(72) Erfinder: **Sitt, Rüdiger, Dr.**
**Claudiusweg 9**
**D-5600 Wuppertal 1(DE)**

(54) **Arzneimittel, die Harnstoffderivate enthalten, Verfahren zu ihrer Herstellung; ein Diphenylharnstoffderivat.**

(57) Verwendung von bekannten und neuen Harnstoffderivaten der allgemeinen Formel I

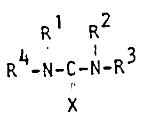
(1)

in welcher

$R^1$ und $R^2$ gleich oder verschieden sind und jeweils für Wasserstoff, geradkettiges, verzweigtes oder cyclisches Alkyl, für Aryl oder Aralkyl stehen, wobei die genannten Alkyl- und Arylreste ihrerseits gegebenenfalls substituiert sind durch Halogen oder Alkoxy,

$R^3$ und $R^4$ gleich oder verschieden sind und jeweils für einen Aryl- oder Heteroarylrest stehen, wobei diese Reste gegebenenfalls durch 1, 2, 3 oder 4 gleiche oder verschiedene Substituenten aus der Gruppe Nitro, Cyano, Halogen, Azido, Hydroxy, Amino, Carboxy, Aminocarbonyl, Aminosulfonyl substituiert sind, wobei die Aminogruppen jeweils durch Alkyl oder Aryl 1- oder 2-fach substituiert sein können, Alkoxycarbonyl, Acyloxy, Acylamino, SO-Alkyl, $SO_2$-Alkyl, Acyl, Phenyl, Phenoxy, Phenylmercapto, Alkyl, Alkoxy oder Alkylmercapto, wobei die genannten Alkyl-, Alkoxy- und Alkylmercaptoreste ihrereseits gegebenenfalls durch 1 oder mehrere Fluoratome substituiert sind und wobei die genannten Phenyl-, Phenylmercapto- und Phenoxysubstituenten ihrerseits wiederum substituiert sein können durch Halogen, Alkyl, Alkoxy oder Alkylmercapto, wobei die Alkyl-, Alkoxy- und Alkylmercaptoreste gegebenenfalls 1 oder mehrfach durch Fluor substituiert sind, oder wobei zwei benachbarte Substituenten am Arylrest gemeinsam mit den beiden Kohlenstoffatomen, an denen sie stehen, für einen gegebenenfalls durch Fluor substituierten Dioxan oder Dioxolring stehen.

X für Sauerstoff, Schwefel oder eine Cyanamidgruppe steht.

Zur Beeinflussung des Fettstoffwechsels sowie Arzneimittel enthaltend diese. Verbindungen.

N(3Chlor-4 trifluormethyl-phenyl)N'-(3-trifluormethyl-phenyl)- harnstoff.

EP 0 022 958 A1

BAYER AKTIENGESELLSCHAFT
Zentralbereich
Patente, Marken und Lizenzen

5090 Leverkusen, Bayerwerk
KS/mo-c
II (Pha)

BEZEICHNUNG GEÄNDERT
siehe Titelseite

Verwendung von Harnstoffderivaten als Arzneimittel bei der Behandlung von Fettstoffwechselstörungen

Die vorliegende Erfindung betrifft die Verwendung von bekannten und neuen Harnstoffderivaten zur Beeinflussung des Fettstoffwechsels sowie Arzneimittel enthaltend diese Verbindungen.

Einige der erfindungsgemäß verwendbaren Harnstoffderivate sind bereits bekannt (vergl. DT-OS 1 443 560; US-Pat. 3 335 142; US-Pat. 3 856 952; US-Pat. 3 903 130). Für diese bekannten Harnstoffderivate sind ebenfalls einige biologische Wirkungen beschrieben. Sie können z.B. als Herbizide, Bakterizide, Fungizide und Futterzusatzstoffe Verwendung finden. Ihre Wirkung auf den Fettstoffwechsel, insbesondere ihre lipidabsorptionshemmende Wirkung ist bisher noch nicht bekannt geworden.

Die vorliegende Erfindung betrifft die Verwendung von Harnstoffderivaten der allgemeinen Formel I

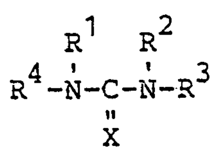

(I)

in welcher

$R^1$ und $R^2$ gleich oder verschieden sind und jeweils für Wasserstoff, geradkettiges, verzweigtes oder cyclisches Alkyl, für Aryl oder Aralkyl stehen, wobei die genannten Alkyl- und Arylreste ihrerseits gegebenenfalls substituiert sind durch Halogen oder Alkoxy,

$R^3$ und $R^4$ gleich oder verschieden sind und jeweils für einen Aryl- oder Heteroarylrest stehen, wobei diese Reste gegebenenfalls durch 1, 2, 3 oder 4 gleiche oder verschiedene Substituenten aus der Gruppe Nitro, Cyano, Halogen, Azido, Hydroxy, Amino, Carboxy, Aminocarbonyl, Aminosulfonyl substituiert sind, wobei die Aminogruppen jeweils durch Alkyl oder Aryl 1- oder 2-fach substituiert sein können, Alkoxycarbonyl, Acyloxy, Acylamino, SO-Alkyl, $SO_2$-Alkyl, Acyl, Phenyl, Phenoxy, Phenylmercapto, Alkyl, Alkoxy oder Alkylmercapto, wobei die genannten Alkyl-, Alkoxy- und Alkylmercaptoreste ihrerseits gegebenenfalls durch 1 oder mehrere Fluoratome substituiert sind und wobei die genannten Phenyl-, Phenylmercapto- und Phenoxysubstituenten ihrerseits wiederum substituiert sein können durch Halogen, Alkyl, Alkoxy oder Alkylmercapto, wobei die Alkyl-, Alkoxy- und Alkylmercaptoreste gegebenenfalls 1 oder mehrfach durch Fluor substituiert sind, oder wobei zwei benachbarte Substituenten am Arylrest gemeinsam mit den beiden Kohlenstoffatomen, an denen sie stehen, für einen gegebenenfalls durch Fluor substituierten Dioxan oder Dioxolring stehen.

X für Sauerstoff, Schwefel oder eine Cyanamidgruppe steht,

bei der Behandlung von Erkrankungen des Fettstoffwechsels sowie bei der Herstellung von fettstoffwechselbeeinflussenden Arzneimitteln, sowie einige neue Verbindungen aus dieser Stoffgruppe.

Überraschenderweise zeigen die Harnstoffderivate der allgemeinen Formel I eine starke lipidabsorptionshemmende Wirkung. Bei Kenntnis des Standes der Technik konnte nicht erwartet werden, daß Verbindungen dieser Stoffklasse als lipidabsorptionshemmende Wirkstoffe verwendet werden können. Ihre bereits bekannte Verwendung als Futterzusatzstoffe in der Tierhaltung und ihre bakterizide Wirkung ließen erwarten, daß nach ihrer Applikation der Körper Nahrungsmittel verstärkt aufnimmt, was zu der in der Tierhaltung erwünschten Gewichtszunahme führt. Die lipidabsorptionshemmende Wirkung und die hieraus resultierende Möglichkeit, die Harnstoffderivate als Zusatzstoffe bei Nahrungsmitteln zu verwenden, bzw. durch Applikation entsprechender Arzneimittelformulierungen die Lipidabsorption aus Nahrungsmitteln zu hemmen, stellt die Überwindung eines aus dem Stand der Technik resultierenden Vorurteils dar.

Die Verwendung von Harnstoffderivaten bei der Behandlung von Hyperlipämien ermöglicht die Behandlung auch solcher Patienten, die gegenüber bereits bekannten Lipidabsorptionshemmern Unverträglichkeit oder Gewöhnung zeigen. Die erstmalige Verwendung der Harnstoffderivate als Wirkstoffe bei der Behandlung von Hyperlipämien stellt somit eine Bereicherung der Pharmazie dar.

Die Harnstoffderivate der allgemeinen Formel I werden in an sich bekannter Weise hergestellt, indem man

a) ein Amin der allgemeinen Formel II

$$R^4-\overset{R^1}{\overset{|}{N}}H \qquad (II)$$

in welcher
$R^1$ und $R^4$ die oben angegebene Bedeutung haben,
mit einer Verbindung der allgemeinen Formel III

$$R^3-NCX \qquad (III)$$

in welcher
$R^3$ und X die oben angegebene Bedeutung haben,
in einem inerten organischen Lösungsmittel bei Temperaturen zwischen 20°C und 120°C umsetzt
[Variante a) ergibt symmetrische und unsymmetrische Harnstoffderivate, in denen $R^2$ immer Wasserstoff bedeutet],

b) oder ein Amin der allgemeinen Formel II

$$R^4-\overset{R^1}{\overset{|}{N}}H \qquad (II)$$

in welcher
$R^1$ und $R^4$ die oben angegebene Bedeutung haben,

mit Chlorameisensäurephenylester der Formel IV

$$ClCOOC_6H_5 \qquad (IV)$$

bei Temperaturen zwischen 0° und 25°C umsetzt und den dabei entstehenden Phenylcarbamidsäureester der allgemeinen Formel V

$$R^4-\overset{\overset{R^1}{|}}{N}-COOC_6H_5 \qquad (V)$$

direkt oder nach Isolierung mit einem Amin der allgemeinen Formel VI

$$R^3-\overset{\overset{R^2}{|}}{N}H \qquad (VI)$$

in welcher
$R^2$ und $R^3$ die oben angegebene Bedeutung haben, in einem inerten organischen Lösungsmittel bei Temperaturen zwischen 20°C und 200°C umsetzt [Variante b) liefert Harnstoffderivate, in denen X Sauerstoff bedeutet],

oder

c) einen Thioester der allgemeinen Formel VII

$$R^4-\overset{\overset{R^1}{|}}{N}-\underset{\underset{X}{\|}}{C}-S\ Alkyl \qquad (VII)$$

in welcher
$R^1$ und $R^4$ die oben angegebene Bedeutung haben,
X für Schwefel oder Cyanamid steht,
mit einem Amin der allgemeinen Formel VI

$$R^3-\overset{\overset{R^2}{|}}{N}H \qquad (VI)$$

in welcher
$R^2$ und $R^3$ die oben angegebene Bedeutung haben,
gegebenenfalls in Gegenwart von inerten organischen Lösungsmitteln bei Temperaturen zwischen 20 und 180°C so lange umsetzt, bis die Alkylmercaptan-Entwicklung beendet ist,
oder

d) eine Verbindung der allgemeinen Formel VIII

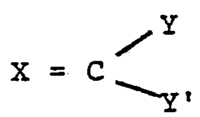

(VIII)

in welcher
X die oben angegebene Bedeutung hat und
Y und Y' gleich oder verschieden sind und für einen nukleophil austauschbaren Rest wie Chlor, Alkylmercapto oder Phenoxy stehen,

mit 2 Mol eines Amins der allgemeinen Formel (II)

$$R^4-\overset{\overset{R^1}{|}}{N}H \qquad (II)$$

in welcher

$R^1$ und $R^4$ die oben angegebene Bedeutung haben, gegebenenfalls in Gegenwart von inerten organischen Lösungsmitteln umsetzt;

[Variante d) ergibt immer symmetrische Harnstoffderivate, in denen jeweils $R^1$ mit $R^2$ und $R^3$ mit $R^4$ identisch sind.]

Bei den vorstehenden Verfahrensvarianten a), b), c) und d) können die Amine der allgemeinen Formel (II) bzw. der allgemeinen Formel (VI) jeweils alternativ eingesetzt werden, wenn die Bedeutung von $R^1$ und $R^4$, bzw. von $R^2$ und $R^3$ in dem jeweiligen Reaktionspartner vorgegeben ist.

Die Erfindung betrifft insbesondere die Verwendung von Harnstoffderivaten der allgemeinen Formel (I),

in welcher

$R^1$ und $R^2$ gleich oder verschieden sind und jeweils für Wasserstoff, geradkettiges oder verzweigtes Alkyl mit 1 bis 4 Kohlenstoffatomen, Phenyl oder Benzyl stehen, wobei die genannten Alkyl-und Phenylreste ihrerseits gegebenenfalls substituiert sind durch Fluor oder Chlor,

$R^3$ und $R^4$ gleich oder verschieden sind und jeweils für einen Phenyl- oder Naphthylrest stehen, wobei diese Reste, insbesondere der Phenylrest, gegebenenfalls substituiert ist durch 1, 2, 3 oder 4 gleiche oder verschiedene Substituenten aus der Gruppe Nitro, Cyano, Halogen, insbesondere Fluor oder Chlor, Azido, Hydroxy, Amino, Carboxy,

Aminocarbonyl, Aminosulfonyl, wobei die Aminogruppen jeweils durch Alkyl mit 1 bis 8 Kohlenstoffatomen oder Phenyl substituiert sein können, Phenyl, Phenoxy, Alkoxycarbonyl mit 1 bis 8 Kohlenstoffatomen, Acyloxy mit 1 bis 8 Kohlenstoffatomen, Acylamido mit 1 bis 8 Kohlenstoffatomen, Acyl mit 1 bis 8 Kohlenstoffatomen, $SO_2$-Alkyl mit 1 bis 8 Kohlenstoffatomen, oder durch Alkyl, Alkoxy oder Alkylmercapto mit jeweils 1 bis 8 Kohlenstoffatomen, wobei diese Alkyl-, Alkoxy- und Alkylmercapto-Reste ihrerseits gegebenenfalls durch Fluor ein- oder mehrfach substituiert sind und

X für Sauerstoff, Schwefel oder die Cyanamidgruppe steht.

Vorzugsweise enthalten die vorgenannten Alkyl-, Alkoxy-, Alkylmercapto- und Acylreste 1 bis 4 Kohlenstoffatome.

Von besonderem Interesse ist die Verwendung von Verbindungen der allgemeinen Formel (I),

in welcher
$R^1$ und $R^2$ jeweils für Wasserstoff stehen,
$R^3$ und $R^4$ gleich oder verschieden sind und jeweils für Phenyl stehen, das durch Halogen, insbesondere Fluor oder Chlor, Trifluormethyl, Trifluormethoxy, Trifluormethylmercapto, Cyano, Carboxy, Alkyl, Alkoxy, Acyl, Alkoxycarbonyl oder Dialkylaminosulfonyl, mit jeweils 1 bis 4 Kohlenstoffatomen in den Alkyl-, Alkoxy- und Acylresten, ein-, zwei-, drei- oder vierfach substituiert ist.

Die Herstellung der neuen Verbindungen aus der durch die allgemeine Formel (I) definierten Stoffklasse erfolgt ebenfalls nach an sich bekannten Methoden gemäß den oben genannten Verfahrensvarianten a) bis c), wobei die als Ausgangsstoffe verwendeten Isocyanatderivate, Amine und Phenylcarbamidsäureester der allgemeinen Formel (III), (IV), (VI) und (VII) bekannt sind oder nach bekannten Methoden hergestellt werden können (vergl. R. Wagner et al, Synthetic Organic Chemistry, Wiley, New York, (1953), S. 640, 645, 653).

Die Harnstoffderivate gemäß Formel (I) zeigen eine vorteilhafte Hemmung der Lipidabsorption bei Mensch und Tier. Bei der Aufnahme fetthaltiger Nahrung führen sie zu einer geringeren alimentären Hyperlipamie, bei gleichzeitiger Hemmung der Cholesterinabsorption, so daß sie insbesondere zur Behandlung von Fettstoffwechselstörungen, wie z. B. Hyperlipoproteinämien, Atherosklerose oder Adipositas verwendet werden können.

Der Nachweis der vorteilhaften Wirkung lässt sich durch folgende Versuchsanordnung an Ratten zeigen:

Zur Erzeugung einer alimentären Hyperlipämie erhält eine Gruppe von Ratten 2,5 ml/kg Olivenöl per os verabreicht (Kontrollgruppe). Eine entsprechende Gruppe von anderen

Ratten erhält gleichzeitig mit der Olivenölapplikation die Wirksubstanz als Suspension in Traganthschleim mit der Schludsonde verabreicht. Eine weitere Kontrollgruppe von Ratten erhält nur Traganthschleim appliziert.

2 Stunden nach der Applikation von Olivenöl werden die Konzentrationen der Serumtriglyceride in allen drei Rattengruppen bestimmt (Methode: J. Ziegenhorst, Klin. Chem. 21, (1975) 1627). Zwei Stunden nach der Fettapplikation zeigen die nur mit Olivenöl behandelten Ratten (Gruppe 1) gegenüber den Ratten ohne Fettapplikation (Gruppe 3) einen deutlichen Anstieg der Serumtriglyceride. Mit diesem Anstieg, der gleich 100 % gesetzt wird, werden die verminderten Serumtriglycerid-Anstiege der mit Wirksubstanz und Olivenöl behandelten Tiere (Gruppe 2) verglichen. Es wurde gefunden, daß bereits geringe Dosierungen der Harnstoffderivate gemäß Formel (I) eine signifikante Senkung der Serumtriglyceride verursachen. Neben der starken lipidabsorptionshemmenden Wirkung zeigen die Verbindungen auch eine ausgesprochen gute Verträglichkeit.

Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen sowie Verfahren zur Herstellung dieser Zubereitungen.

Zur vorliegenden Erfindung gehören auch pharmazeutische Zubereitungen in Dosierungseinheiten. Dies bedeutet, daß

die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Lösungen, Suspensionen und Emulsionen und Pasten genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel,

z.B. Talkum-, Calcium- und Magnesiumstearat und feste Polyäthylenglykole oder Gemische der unter (a) - (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Lösungen und Emulsionen können neben dem oder den Wirkstoffen die üblichen Trägerstoffe, wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Aethylalkohol, Propylenglykol, Suspendiermittel, z.B. äthoxylierte Isostearylalkohole, Polyoxyäthylensorbit- und -sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl, und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gewichtsprozent der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen der oben angegebenen Formel enthalten, in der

Human- und Veterinärmedizin zur Verhütung, Besserung und/oder Heilung von Erkrankungen des Fettstoffwechsels.

Die Wirkstoffe oder die pharmazeutischen Zubereitungen können oral oder parenteral, vorzugsweise oral, appliziert werden.

Im allgemeinen hat es sich sowohl in der Human- als auch in der Veterinär-Medizin als vorteilhaft erwiesen, den oder die Wirkstoffe in Mengen von etwa 1,0 bis etwa 500, vorzugsweise 5 bis 100 mg/kg Körpergewicht je 24 Stunden, verteilt auf 1 bis 6 Verabreichungen, und zwar vor und/oder während und/oder nach der Mahlzeit zu applizieren. Eine Einzelgabe enthält den oder die Wirkstoffe vorzugsweise in Mengen von etwa 1 bis etwa 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die folgenden Formulierungsbeispiele erläutern die Herstellung von erfindungsgemäß zu verwendenden Arzneimittelzubereitungen:

Beispiele für Tablettenherstellung

1. 100 mg der Verbindung des Beispiels 1 werden mit 69 mg Milchzucker und 30 mg Maisstärke gemischt, anschließend mit einem Kleister aus 15 mg Maisstärke angeknetet und durch ein Sieb mit 3 - 5 mm Maschenweite gedrückt. Anschließend wird in einem Trockner bei 60 - 80°C getrocknet.
   Das erhaltene Granulat wird durch ein Sieb mit 0,8 mm Maschenweite geschlagen, weitere 15 mg Maisstärke, 10 mg Kalkum und 1 mg Magnesiumstearat werden zugemischt und mit Hilfe einer üblichen Tablettenpresse zu runden Tabletten mit 9 mm Durchmesser und einem Gesamtgewicht von 240 mg verpreßt.

2. 200 mg der Verbindung des Beispiels 29 werden mit 97 mg sekundärem Calciumphosphat vermischt und mit einer wässrigen Gelantinelösung, die 2 mg Gelantine enthält angeknetet. Anschließend wird durch ein Sieb mit 3 - 5 mm Maschenweite gedrückt und bei 60 - 80°C getrocknet. Das trockene Granulat wird gesiebt (0,8 mm), anschließend 20 mg Weizenstärke und 1 mg Magnesiumstearat zugemischt und auf bekannte Weise tablettiert. Man erhält runde Tabletten vom Durchmesser 8 mm und einem Gesamtgewicht von 320 mg.

Besonders geeignet sind Harnstoffderivate der allgemeinen Formel (I),

in der

$R^1$ und $R^2$ für Wasserstoff stehen,

X für Sauerstoff steht und

$R^3$ und $R^4$ jeweils für Phenyl stehen, welches durch 1, 2 oder 3 gleiche oder verschiedene Substituenten aus der Gruppe Chlor, Fluor, Cyano, Trifluormethyl, Trifluormethoxy, Trifluormethylmercapto, Dialkylaminosulphonyl, Alkoxycarbonyl oder Alkyl, mit jeweils 1 bis 4 Kohlenstoffatomen in den Alkyl- und Alkoxyresten, substituiert ist.

Die nachfolgenden Beispiele erläutern die Herstellung der erfindungsgemäß verwendbaren Wirkstoffe:

Bemerkung: In den nachfolgenden Beispielen besitzen die folgenden Abkürzungen folgende Bedeutung:

| | | |
|---|---|---|
| THF | = | Tetrahydrofuran |
| DMSO | = | Dimethylsulfoxid |
| Tol | = | Toluol |

Beispiel 1 (Variante a)

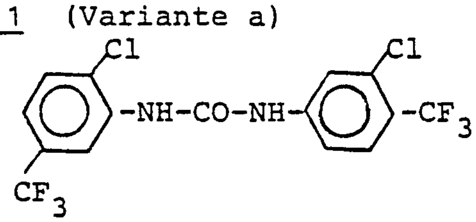

0,05 Mol 2-Chlor-5-trifluormethylanilin werden in 30 ml Tetrahydrofuran gelöst und mit einer Lösung von 0,05 Mol 3-Chlor-4-trifluormethylphenylisocyanat in 30 ml Tetrahydrofuran verrührt. Das Reaktionsgemisch wird auf 50°C erwärmt, wobei der N-2-Chlor-5-trifluormethylphenyl-N'-3-chlor-4-trifluormethylphenyl-harnstoff ausfällt. Schmelzpunkt 210-212°C; Ausbeute: 82 % der Theorie.

Beispiel 2 (Variante b)

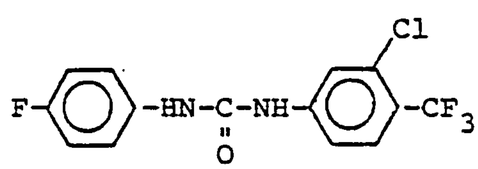

Eine Lösung von 0,5 Mol p-Fluoranilin in 300 ml Dichlorbenzol und 70 ml (0,5 Mol) Triäthylamin wird mit 0,5 Mol Chlorameisensäurephenylester unter Kühlung (0-10°C) versetzt. Das Reaktionsgemisch wird anschließend 24 Stunden bei Raumtemperatur stehengelassen. Man saugt vom entstandenen Triethylamin-hydrochlorid ab und versetzt das Filtrat mit 0,5 Mol 3-Chlor-4-trifluormethylanilin. Die Reaktionslösung wird 6 Stunden auf 180°C erhitzt, das Lösungsmittel unter Vakuum abgezogen und der Rückstand in 250 ml Ether aufgekocht und erneut abgesaugt. Man erhält N-4-Fluorphenyl-N'-3-chlor-4-trifluormethylphenylharnstoff vom Schmelzpunkt 212 bis 214°C. Ausbeute: 67 % der Theorie.

Beispiel 3 (Variante c)

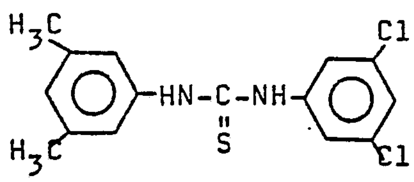

Eine Lösung von 0,2 Mol 3,5-Dichloranilin in 150 ml Tetrahydrofuran wird mit 30 ml Triäthylamin versetzt. Man tropft 0,25 Mol Schwefelkohlenstoff zu, erhitzt 5 Stunden auf 50 - 60°C, kühlt auf 20°C ab und versetzt anschließend mit 0,25 Mol Methyljodid. Nach 2-stündigem Rühren bei 20 - 25°C werden 200 ml Xylol zugesetzt und die organische Phase mit Wasser ausgeschüttelt, anschließend eingeengt und mit 0,2 Mol 3,5-Dimethylanilin versetzt. Dann wird das Reaktionsgemisch bis zum Nachlassen der Methylmercaptanentwicklung auf 80 - 140°C erhitzt. Man erhält N-3,5-Dichlorphenyl-N'-3,5-dimethylphenyl-thioharnstoff, der nach dem Abkühlen abgesaugt wird.
Schmelzpunkt: 265 - 267°C; Ausbeute: 49 % der Theorie.

Beispiel 4 (Variante d)

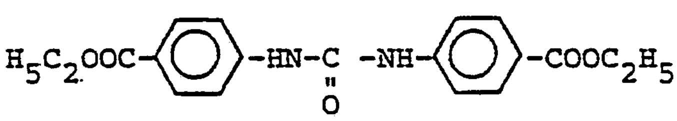

Ein Gemisch von 0,2 Mol p-Aminobenzoesäureethylester, 150 ml 3,5-Dichlorbenzol und 0,1 Mol Diphenylcarbonat wird 6 Stunden auf 180°C erhitzt. Die Reaktionsmischung wird abgekühlt und nach 24 Stunden abgesaugt. Man erhält Bis-4-ethoxycarbonylphenyl-harnstoff.
Schmelzpunkt: 223-225°C; Ausbeute 62 % der Theorie.

Beispiel 5 (Variante a)

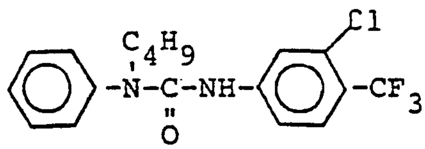

0,05 Mol N-Butylanilin werden in 200 ml Methylenchlorid mit 0,05 Mol 4-Trifluormethyl-3-chlorphenylisocyanat bei Raumtemperatur umgesetzt. Nach 12 Stunden wird der ausgefallene Niederschlag isoliert und aus Methanol umkristallisiert. Man erhält N-4-Trifluormethyl-3-chlorphenyl-N'-butyl-N'-phenylharnstoff.
Schmelzpunkt: 95°C; Ausbeute: 75 % der Theorie.

Beispiel 6 (Variante a)

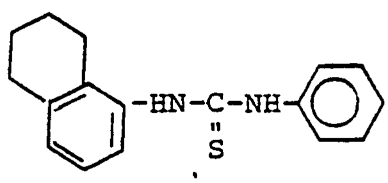

0,1 Mol 5-Aminotetralin werden in 30 ml Toluol gelöst und mit 0,1 Mol Phenylsenföl versetzt, wobei die Temperatur auf 35°C ansteigt und sich ein Niederschlag bildet. Man läßt 24 Stunden stehen und saugt den N-5-Tetralyl-N'-phenyl-thioharnstoff ab.
Schmelzpunkt: 145-147°C; Ausbeute: 83 % der Theorie.

Die folgenden Tabellenbeispiele werden, wenn nicht ausdrücklich anders angegeben, gemäß Variante a) analog Beispiel 1 hergestellt:

Tabelle 1

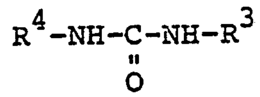 (I a)

| Bei-spiel Nr. | $R^4$ | $R^3$ | Fp(°C) | Lösungs-mittel | Aus-beute (% der Theorie) |
|---|---|---|---|---|---|
| 7) | 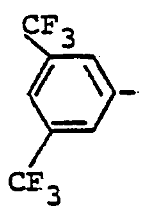 | 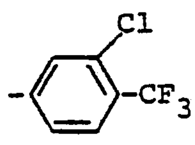 | 202-203 | Tol | 72 |
| 8) | 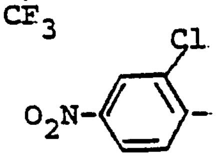 | 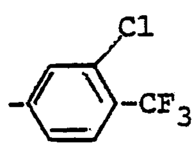 | 125-128 | THF | 91 |
| 9) | 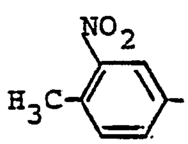 | 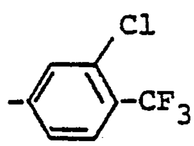 | 243-244 | THF | 64 |
| 10) | 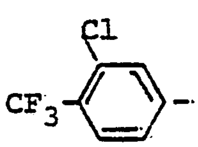 | 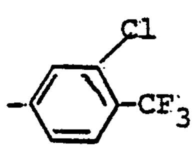 | 263-264 | Tol | 92 |
| 11) | 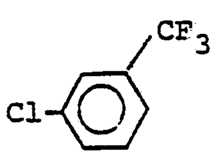 | 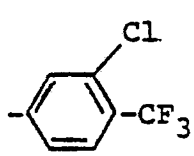 | 208-210 | Tol | 58 |
| 12) | 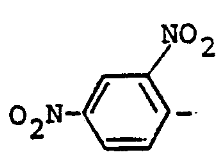 | 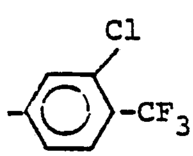 | 164 | DMSO | 51 |

| Beispiel Nr. | $R^4$ | $R^3$ | Fp(°C) | Lösungsmittel | Ausbeute (% der Theorie) |
|---|---|---|---|---|---|
| 13) | 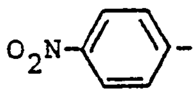 | 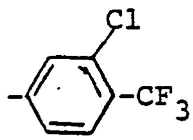 | 221 | THF | 87 |
| 14) | 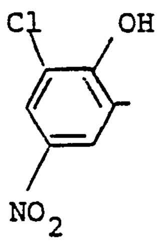 | 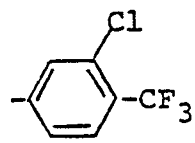 | 135-136 | THF | 70 |
| 15) | 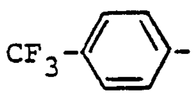 | 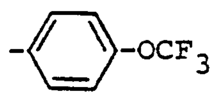 | 208-210 | Tol | 79 |
| 16) | 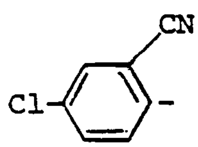 | 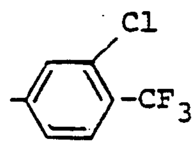 | 236-238 | THF | 74 |
| 17) | 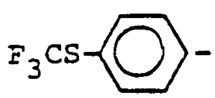 | 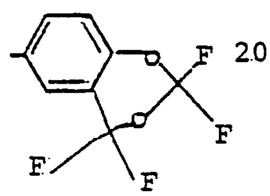 | 205-207 | Tol | 91 |
| 18) | 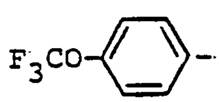 | 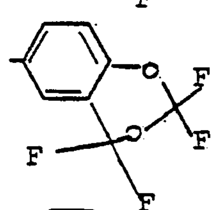 | 173-175 | Tol | 93 |
| 19) | 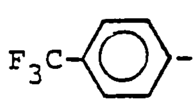 | 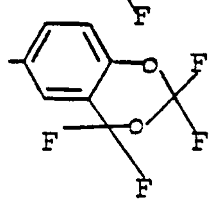 | 200-202 | Tol | 95 |
| 20) | 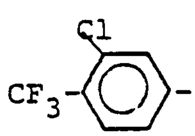 | 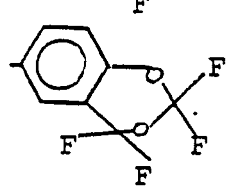 | 249-251 | Tol | 90 |

| Beispiel Nr. | $R^4$ | $R^3$ | Fp(°C) | Lösungsmittel | Ausbeute (% der Theorie) |
|---|---|---|---|---|---|
| 21) | 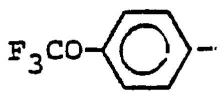 | 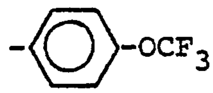 | 215-217 | Tol | 78 |
| 22) | 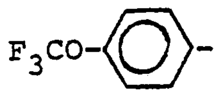 | 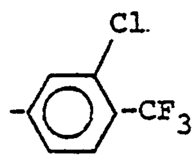 | 189-191 | Tol | 87 |
| 23) | 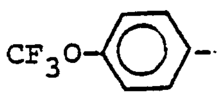 | 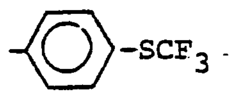 | 239-241 | Tol | 96 |
| 24) | 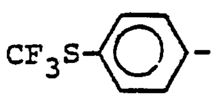 | 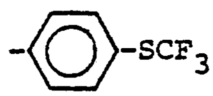 | 267-269 | Tol | 72 |
| 25) | 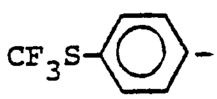 | 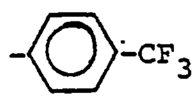 | 149-151 | Tol | 68 |
| 26) | 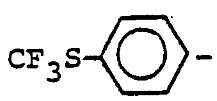 | 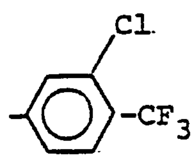 | 129-131 | Tol | 96 |
| 27) | 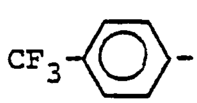 | 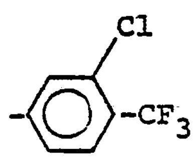 | 245-247 | Tol | 98 |
| 28) | 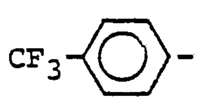 | 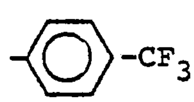 | 137-139 | Tol | 89 |

| Beispiel Nr. | $R^4$ | $R^3$ | Fp(°C) | Lösungsmittel | Ausbeute (% der Theorie) |
|---|---|---|---|---|---|
| 29) | 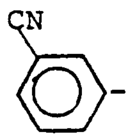 | 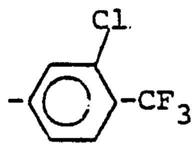 | 230-234 | THF | 89 |
| 30) | 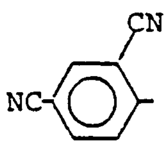 | 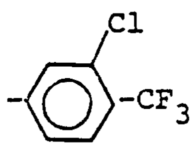 | 167-169 | THF | 81 |
| 31) | 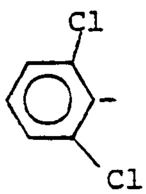 | 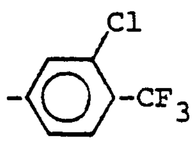 | 243-245 | THF | 64 |
| 32) | 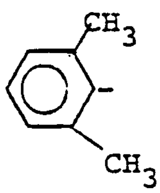 | 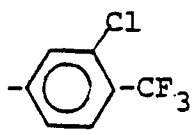 | 235-237 | Tol | 75 |
| 33) | 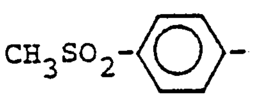 | 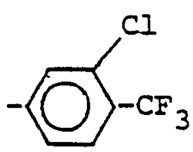 | 224-226 | DMSO | 61 |
| 34) | 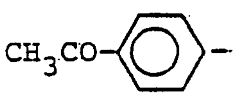 | 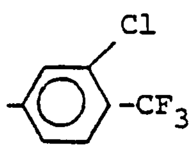 | 222-225 | THF | 84 |
| 35) | 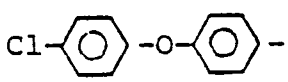 | 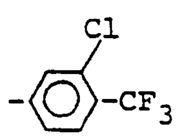 | 215-217 | Tol | 60 |

| Beispiel Nr. | $R^4$ | $R^3$ | Fp(°C) | Lösungsmittel | Ausbeute (% der Theorie) |
|---|---|---|---|---|---|
| 36) | 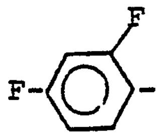 | 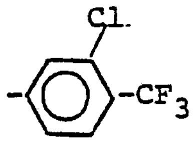 | 212-214 | Tol | 86 |
| 37) | 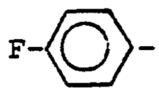 | 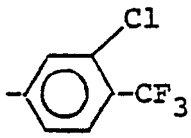 | 212-214 | Tol | 76 |
| 38) | 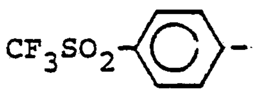 | 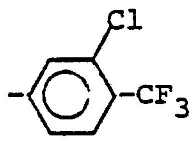 | 245-248 | DMSO | 71 |
| 39) | 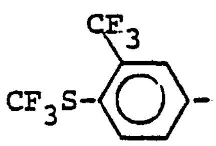 | 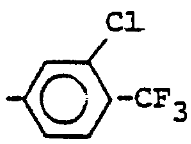 | 197-199 | THF | 82 |
| 40) | 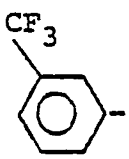 | 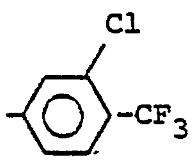 | 184-186 | Tol | 69 |
| 41) | 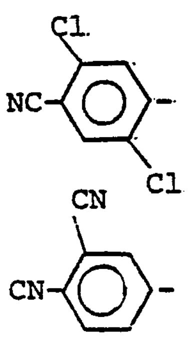 | 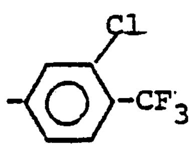 | 255-257 | THF | 70 |
| 42) | | 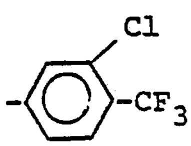 | 273-275 | THF | 81 |

| Beispiel Nr. | $R^4$ | $R^3$ | Fp(°C) | Lösungsmittel | Ausbeute (% der Theorie) |
|---|---|---|---|---|---|
| 43) | 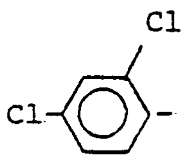 | 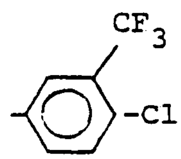 | 238-240 | Tol | 96 |
| 44) | 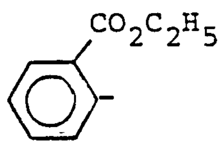 | 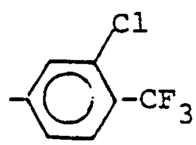 | 188-190 | Tol | 88 |
| 45) | 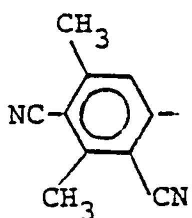 | 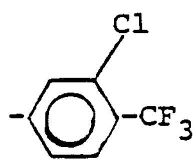 | 190-200 | THF | 48 |
| 46) | 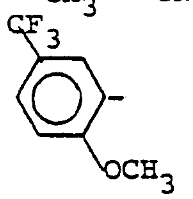 | 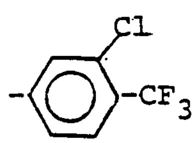 | 215-217 | THF | 62 |
| 47) | 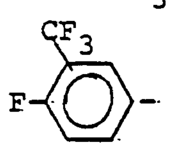 | 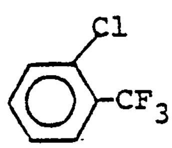 | 218-220 | THF | 93 |
| 48) | 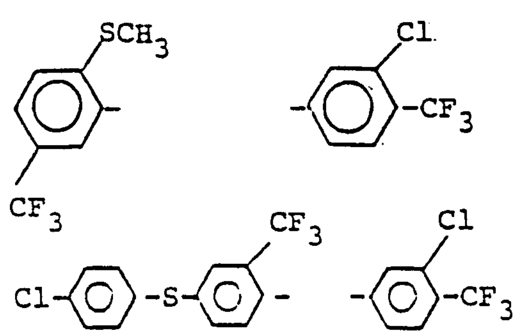 | | 187-189 | THF | 78 |
| 49) | | | 202-204 | THF | 42 |

| Beispiel Nr. | $R^4$ | $R^3$ | Fp(°C) | Lösungsmittel | Ausbeute (% der Theorie) |
|---|---|---|---|---|---|
| 50) | 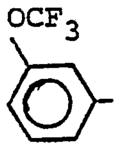 | 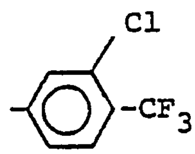 | 143-46 | Tol | 86 |
| 51) | 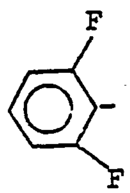 | 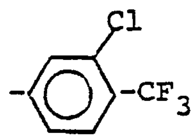 | 210-212 | THF | 54 |
| 52) | 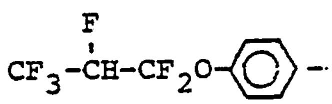 | 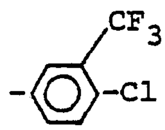 | 140-143 | THF | 68 |
| 53) | 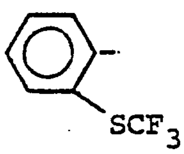 | 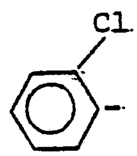 | 194-196 | THF | 74 |
| 54) | 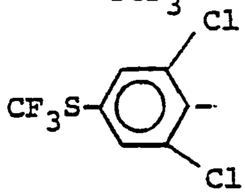 | 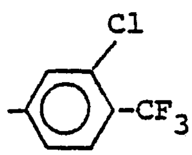 | 198-200 | THF | 82 |
| 55) | 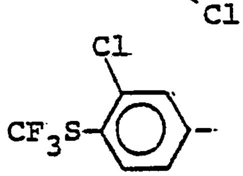 | 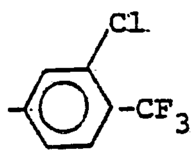 | 217-219 | Tol | 88 |
| 56) | 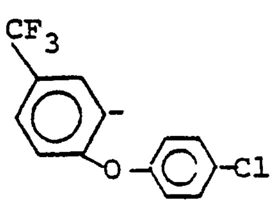 | 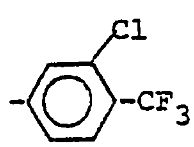 | 220-221 | THF | 62 |

| Beispiel Nr. | $R^4$ | $R^3$ | Fp(°C) | Lösungsmittel | Ausbeute (% der Theorie) |
|---|---|---|---|---|---|
| 57) | 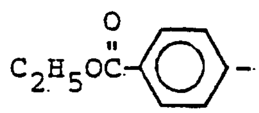 | 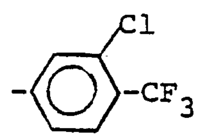 | 234-237 | Tol | 84 |
| 58) | 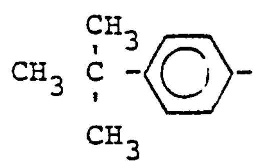 | 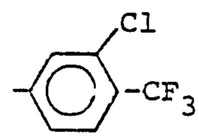 | 234 | Tol | 92 |
| 59) | 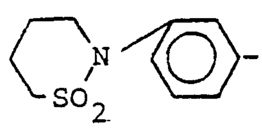 | 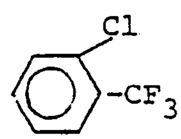 | 223 | DMSO | 59 |
| 60) | 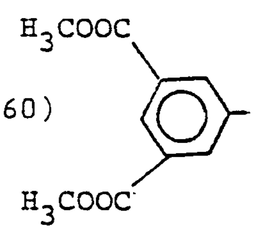 | 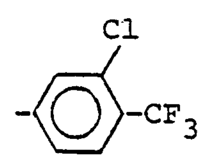 | 220 | THF | 85 |
| 61) | 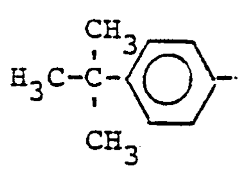 | 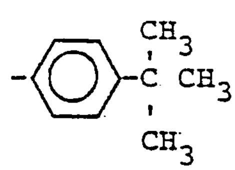 | 298 | Xylol | 71 |
| 62) | 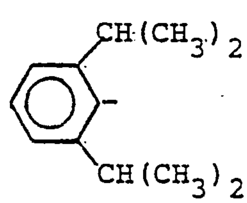 | 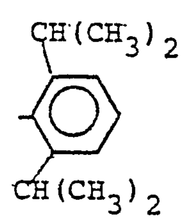 | 230 | Dichlorbenzol | 49 |
| 63) | 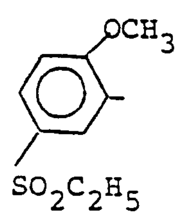 | 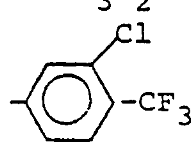 | 254 | DMSO | 78 |

| Bei-spiel Nr. | $R^4$ | $R^3$ | Fp(°C) | Lösungs-mittel | Aus-beute (% der Theorie) |
|---|---|---|---|---|---|
| 64) | 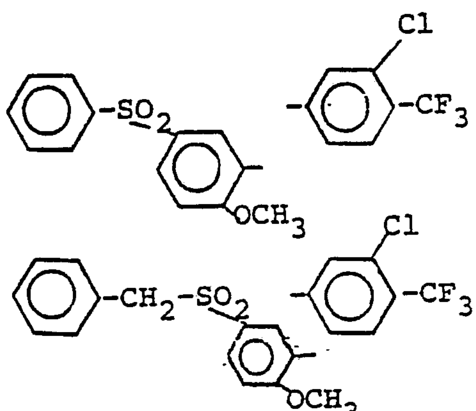 | | 258 | DMSO | 80 |
| 65) | | | 231 | THF | 72 |
| 66) | 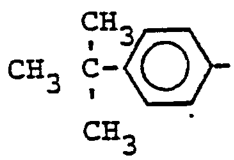 | 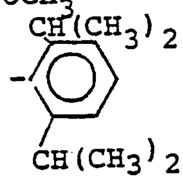 | 246 | THF | 89 |
| 67) | 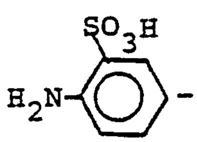 | 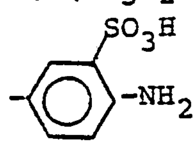 | | | |
| 68) | 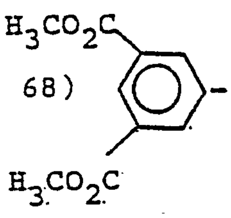 | 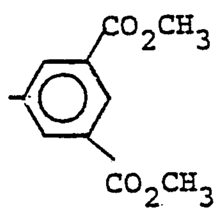 | 259 | Dichlor-benzol | 54 |
| 69) | 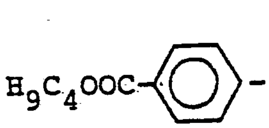 | 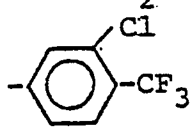 | 224 | THF | 88 |
| 70) | 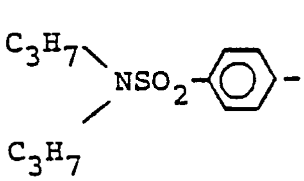 | 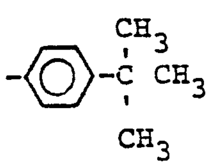 | 197 | THF | 84 |

| Beispiel Nr. | $R^4$ | $R^3$ | Fp(°C) | Lösungsmittel | Ausbeute (% der Theorie) |
| --- | --- | --- | --- | --- | --- |
| 71) | 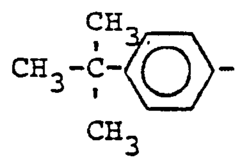 | 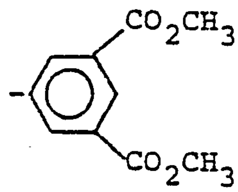 | 216 | Tol | 66 |
| 72) | 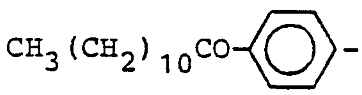 | 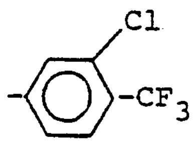 | 185 | Tol | 74 |
| 73) | 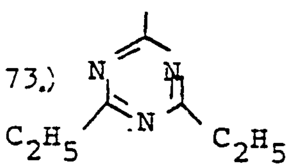 | 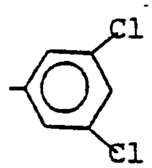 | 191-194 | THF | 92 |
| 74) | 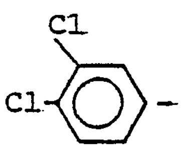 | 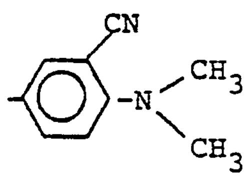 | 193-196 | THF | 80 |
| 75) | 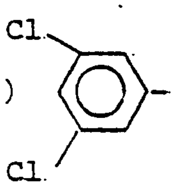 | 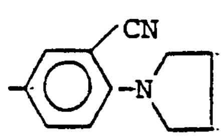 | 226-228 | THF | 76 |
| 76) | 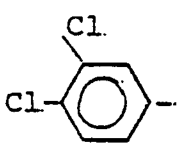 | 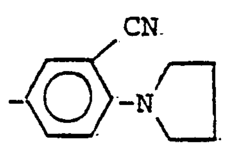 | 222-224 | THF | 79 |
| 77) | 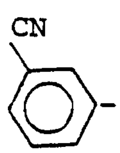 | 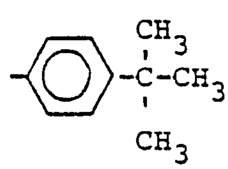 | 178 | Tol | 86 |

| Beispiel Nr. | $R^4$ | $R^3$ | Fp(°C) | Lösungsmittel | Ausbeute (% der Theorie) |
|---|---|---|---|---|---|
| 78) | 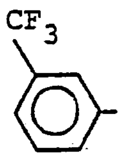 | 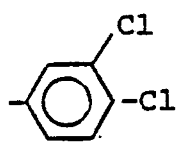 | 193 | Tol | 79 |
| 79) | 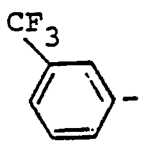 | 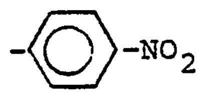 | | THF | 92 |
| 80) | 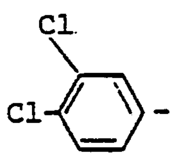 | 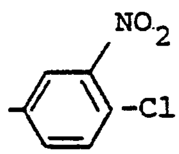 | 253 | THF | 95 |
| 81) | 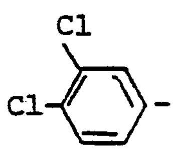 | 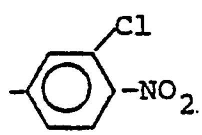 | 276 | THF | 89 |
| 82) | 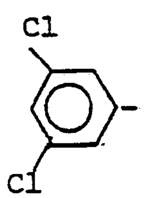 | 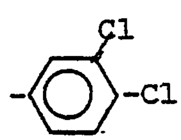 | 249 | Tol | 95 |
| 83) | 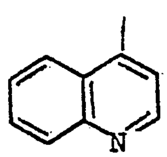 | 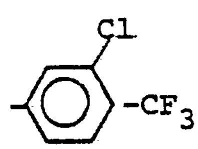 | 119-121 | THF | 88 |
| 84) | 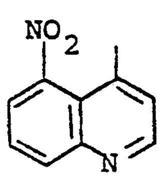 | 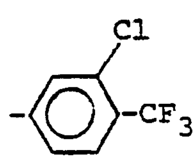 | 298-300 | DMSO | 76 |

| Beispiel Nr. | $R^4$ | $R^3$ | Fp(°C) | Lösungsmittel | Ausbeute (% der Theorie) |
|---|---|---|---|---|---|
| 85) | 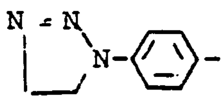 | 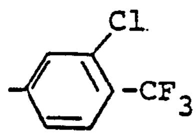 | 244-247 | THF | 63 |
| 86) | 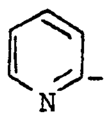 | 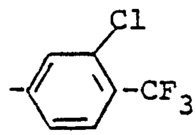 | 208-310 | THF | 86 |
| 87) | 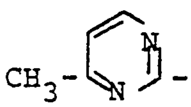 | 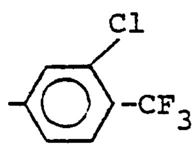 | 230-231 | THF | 77 |
| 88) | 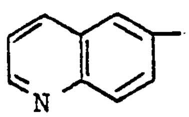 | 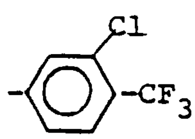 | 258-260 | THF | 81 |
| 89) | 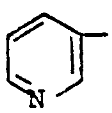 | 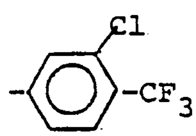 | 222-224 | THF | 68 |
| 90) | 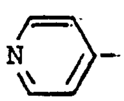 | 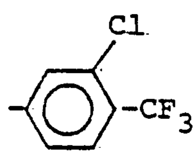 | 168-171 | THF | 75 |
| 91) | 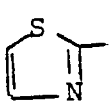 | 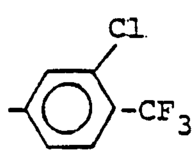 | 179 | DMSO | 65 |

| Beispiel Nr. | $R^4$ | $R^3$ | Fp(°C) | Lösungsmittel | Ausbeute (% der Theorie) |
|---|---|---|---|---|---|
| 92) | 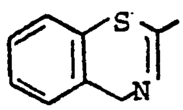 | 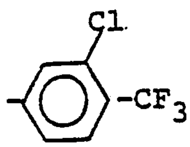 | 270 | DMSO | 49 |
| 93) | 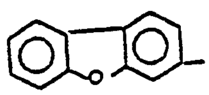 | 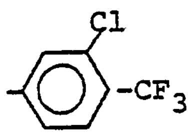 | 270 | THF | 91 |
| 94) | 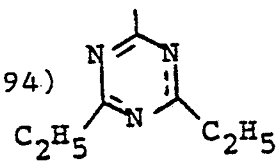 | 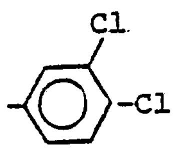 | 193-195 | THF | 84 |
| 95) | 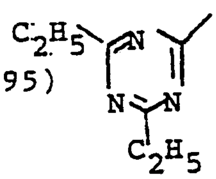 | 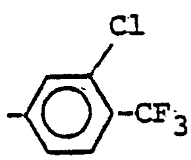 | 194-195 | THF | 89 |
| 96) | 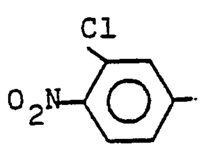 | 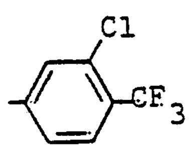 | 123-125 | THF | 83 |
| 97) | 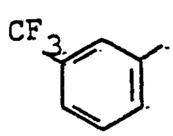 | 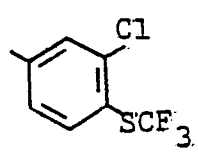 | 152-153 | THF | 88 |
| 98) | 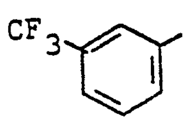 | 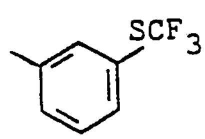 | 168-170 | THF | 81 |

| Beispiel Nr. | $R^4$ | $R^3$ | Fp(°C) | Lösungsmittel | Ausbeute (% der Theorie) |
|---|---|---|---|---|---|
| 99) | 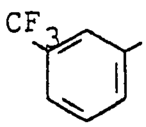 | 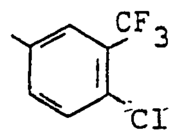 | 173-175 | THF | 69 |
| 100) | 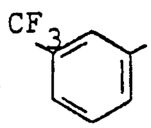 | 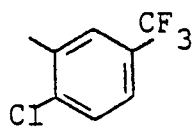 | 161-164 | THF | 72 |
| 101) | 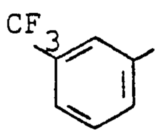 | 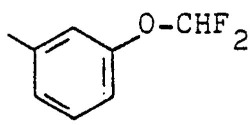 | 146-147 | THF | 90 |
| 102) | 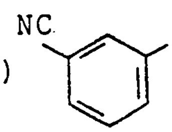 | 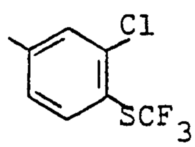 | 225-227 | THF | 93 |
| 103) | 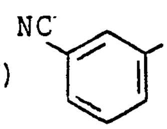 | 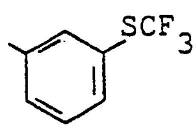 | 212-214 | THF | 79 |
| 104) | 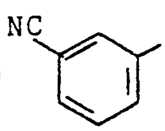 | 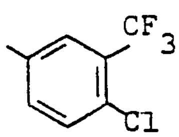 | 200-202 | THF | 81 |
| 105) | 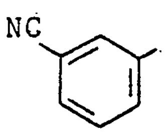 | 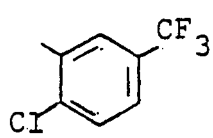 | 213-215 | THF | 84 |
| 106) | 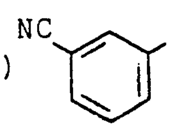 | 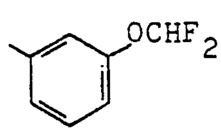 | 116-118 | THF | 90 |

| Beispiel Nr. | $R^4$ | $R^3$ | Fp($^oC$) | Lösungsmittel | Ausbeute (% der Theorie) |
|---|---|---|---|---|---|
| 107) | 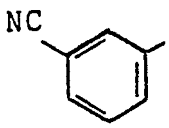 | 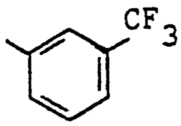 | 159-161 | THF | 92 |
| 108) | 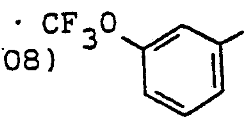 | 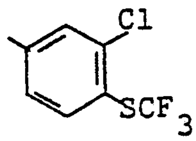 | 129-131 | THF | 91 |
| 109) | 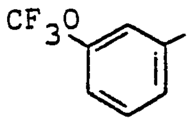 | 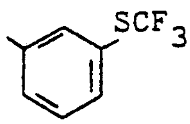 | 124-126 | THF | 72 |
| 110) | 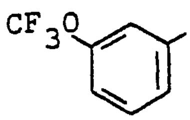 | 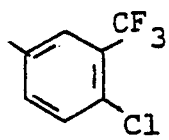 | 128-131 | THF | 89 |
| 111) | 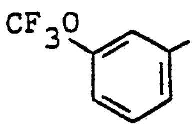 | 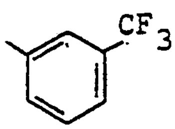 | 168-170 | THF | 82 |
| 112) | 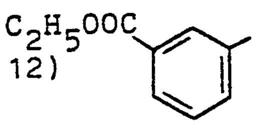 | 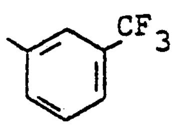 | 135-137 | THF | 88 |
| 113) | 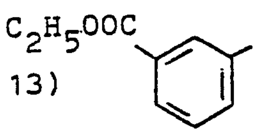 | 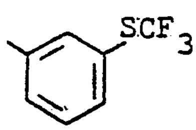 | 120-121 | THF | 83 |
| 114) | 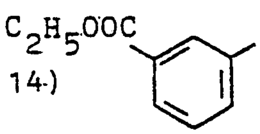 | 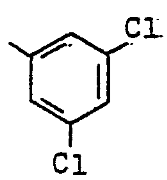 | 213-215 | THF | 72 |
| 115) | 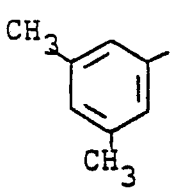 | 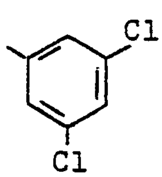 | 265-267 | THF | 89 |

| Beispiel Nr. | $R^4$ | $R^3$ | Fp($^oC$) | Lösungsmittel | Ausbeute (% der Theorie) |
|---|---|---|---|---|---|
| 116) | 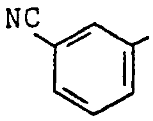 | 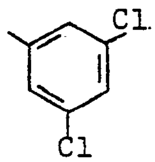 | 220-222 | THF | 87 |
| 117) | 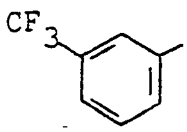 | 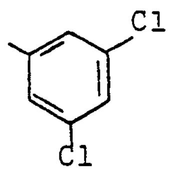 | 180-182 | THF | 78 |
| 118) | 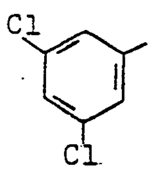 | 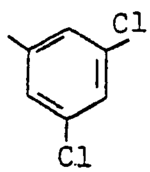 | 296-298 | THF | 75 |
| 119) | 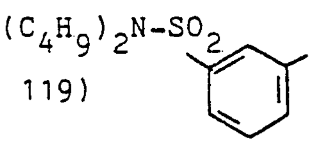 | 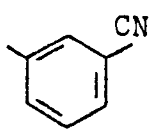 | 105-109 | THF | 64 |
| 120) | 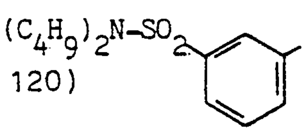 | 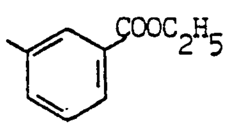 | 108-110 | THF | 91 |
| 121) | 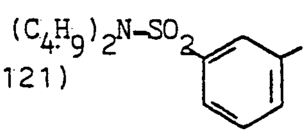 | 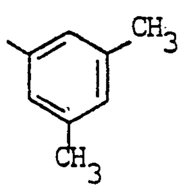 | 106-108 | THF | 79 |

| Beispiel Nr. | $R^4$ | $R^3$ | Fp(°C) | Lösungsmittel | Ausbeute (% der Theorie) |
|---|---|---|---|---|---|
| 121a | 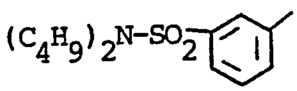 | 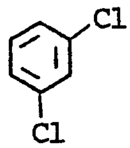 | 165-7° | THF | 94 |
| b | 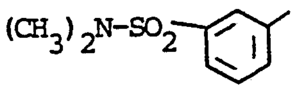 | 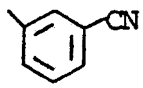 | 181-3° | THF | 96 |
| b' | " | 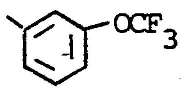 | 173-5° | THF | 84 |
| c | " | 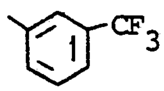 | 164-6° | THF | 88 |
| c' | " | 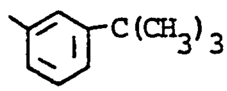 | 184-6° | THF | 81 |
| d | " | 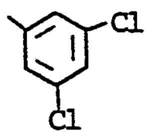 | 198-200° | THF | 83 |
| e | 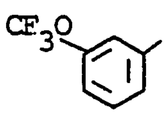 | 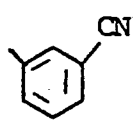 | 167-9° | THF | 78 |
| f | 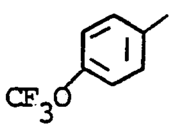 | 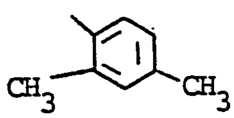 | 211-3° | THF | 81 |
| g | 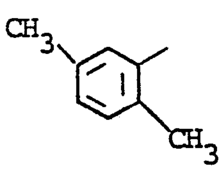 | 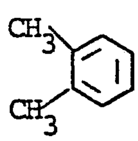 | 221-3° | THF | 84 |

| Beispiel Nr. | $R^4$ | $R^3$ | Fp(°C) | Lösungsmittel | Ausbeute (% der Theorie) |
|---|---|---|---|---|---|
| h | 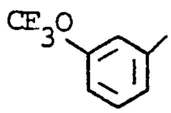 | 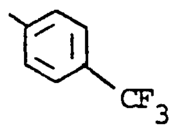 | 192-4° | THF | 73 |
| j | " | 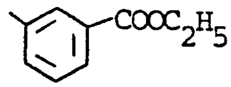 | 139-141° | THF | 90 |
| k | 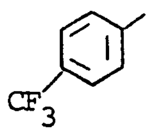 | 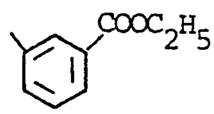 | 173-5° | THF | 81 |
| l | " | 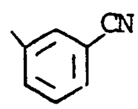 | 216-8° | THF | 95 |
| m | 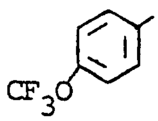 | 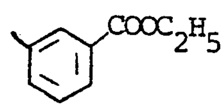 | 163-5° | THF | 79 |
| n | " | 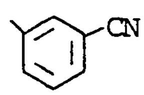 | 178-180° | THF | 99 |
| o | " | 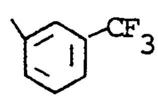 | 143-5° | THF | 79 |
| p | 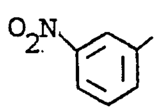 | 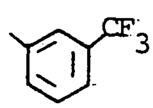 | 203-5° | THF | 76 |
| q | 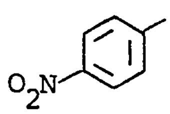 | 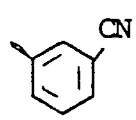 | 238-40° | Tol | 51 |

Tabelle 2

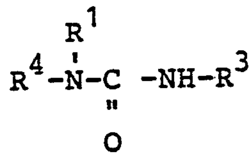 (I b)

| Bei-spiel Nr. | $R^1$ | $R^4$ | $R^3$ | Fp | Lösungs-mittel | Aus-beute (% der Theorie) |
|---|---|---|---|---|---|---|
| 122) | 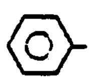 | 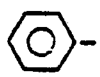 | 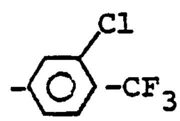 | 117 | Tol | 81 |
| 123) | 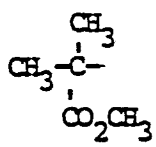 | 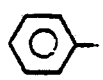 | 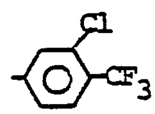 | 121 | Tol | 85 |
| 124) | $CH_3$- | 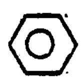 | 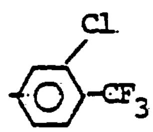 | 95 | Tol | 68 |
| 125) | $CH_3$- | 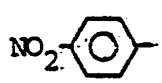 | 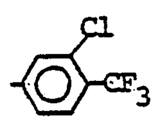 | 187 | Tol | 92 |
| 126) | $C_4H_9$- | 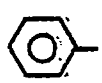 | 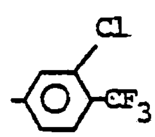 | 97 | Tol | 79 |
| 127) | 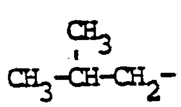 | 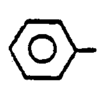 | 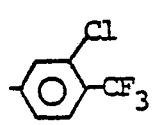 | 101 | Tol | 86 |

| Beispiel Nr. | $R^1$ | $R^4$ | $R^3$ | Fp | Lösungsmittel | Ausbeute (% der Theorie) |
|---|---|---|---|---|---|---|
| 128) | 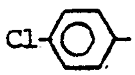 | 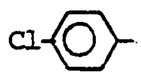 | 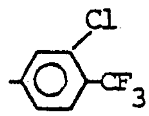 | 225 | THF | 87 |
| 129) | $CH_3$ | 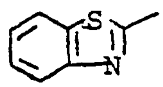 | 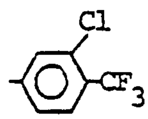 | 153 | THF | 70 |

Tabelle 3

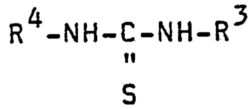

| Beispiel Nr. | $R^4$ | $R^3$ | Fp($^oC$) | Lösungsmittel | Ausbeute (% der Theorie) |
|---|---|---|---|---|---|
| 130) | 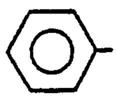 | 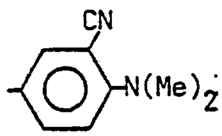 | 138-139°C | THF | 82 |
| 131) | 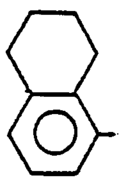 | 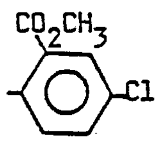 | 152-154°C | THF | 71 |
| 132) | 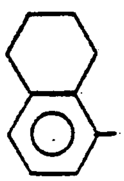 | 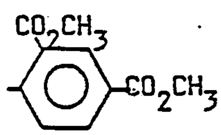 | 171-175°C | THF | 79 |
| 133) | 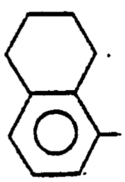 | 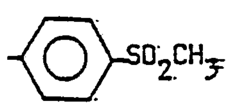 | 167-170°C | THF | 84 |
| 134 | 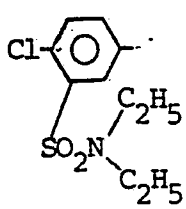 | 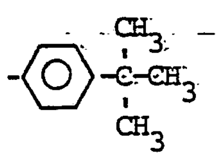 | 115-117° | Toluol | 64 |

| Beispiel Nr. | $R^4$ | $R^3$ | Fp(°C) | Lösungsmittel | Ausbeute (% der Theorie) |
|---|---|---|---|---|---|
| 135 | 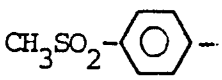 | 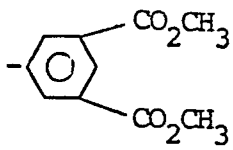 | 251°C | Toluol | 72 |
| 136 | 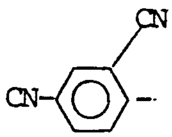 | 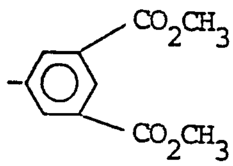 | 190°C | Toluol | 71 |
| 137 | 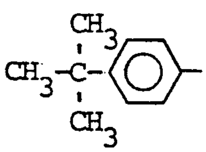 | 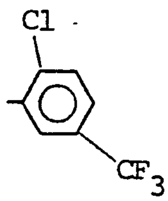 | 182-4° | Toluol | 83 |
| 138 | 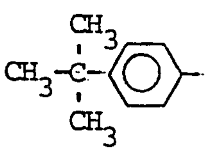 | 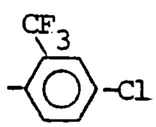 | 167°C | Toluol | 76 |
| 139 | 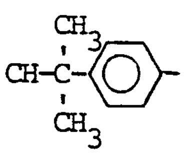 | 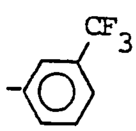 | 194°C | Toluol | 72 |
| 140 | 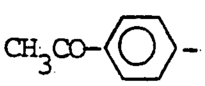 | 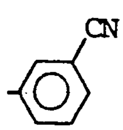 | 192°C | Toluol | 68 |
| 141 | 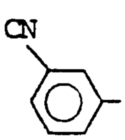 | 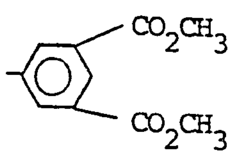 | 209°C | Toluol | 77 |

| Beispiel Nr. | $R^4$ | $R^3$ | Fp(°C) | Lösungsmittel | Ausbeute (% der Theorie) |
|---|---|---|---|---|---|
| 142 | 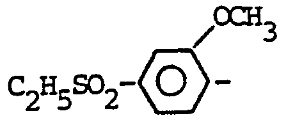 | 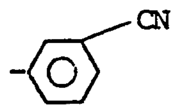 | 211°C | Toluol | 78 |
| 143 | 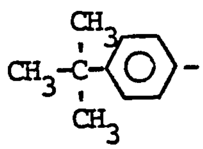 | 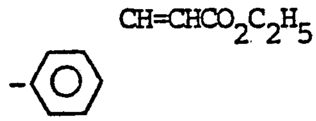 | 198°C | Toluol | 61 |
| 144 | 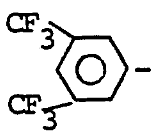 | 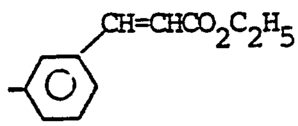 | 205°C | Toluol | 56 |
| 145 | 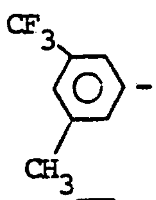 | 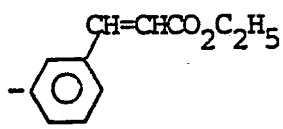 | 178°C | Toluol | 58 |
| 146 | 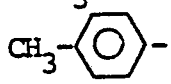 | 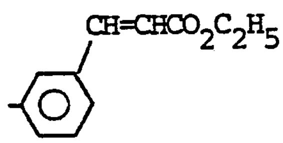 | 168°C | Toluol | 62 |
| 147 | 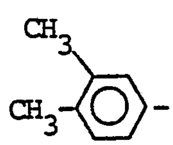 | 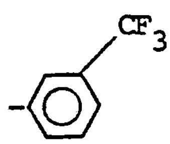 | 181°C | Toluol | 84 |
| 148 | 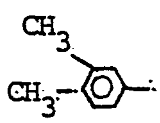 | 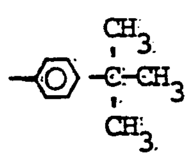 | 201°C | Toluol | 71 |
| 149 | 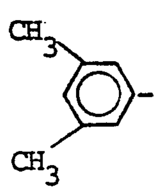 | 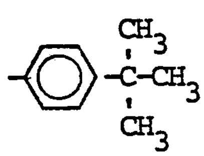 | 231°C | Toluol | 79 |

| Bei-spiel Nr. | $R^4$ | $R^3$ | Fp($^oC$) | Lösungs-mittel | Ausbeute (% der Theorie) |
|---|---|---|---|---|---|
| 150 | 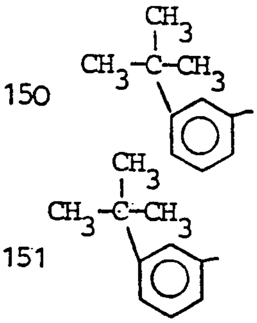 | 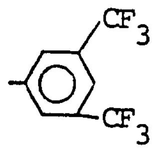 | 172°C | Toluol | 81 |
| 151 | | 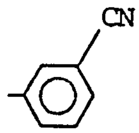 | 171°C | Toluol | 82 |
| 152 | 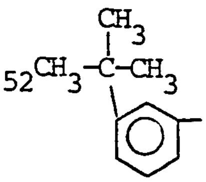 | 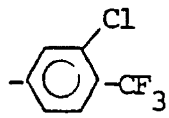 | 151°C | Toluol | 87 |
| 153 | 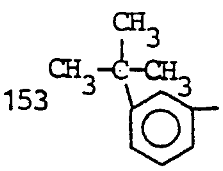 | 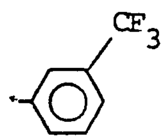 | 151°C | Toluol | 70 |
| 154 | 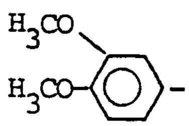 | 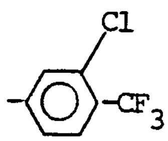 | 197°C | Chloroform | 68 |
| 155 | 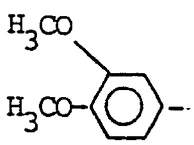 | 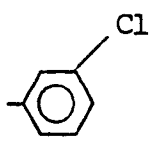 | 194°C | Chloroform | 81 |
| 156 | 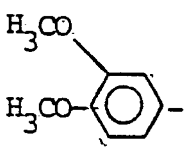 | 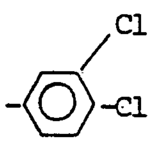 | 195°C | Chloroform | 63 |
| 157 | 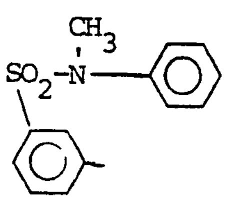 | 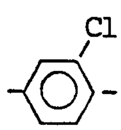 | 166°C | Chloroform | 73 |

| Beispiel Nr. | $R^4$ | $R^3$ | Fp(°C) | Lösungsmittel | Ausbeute (% der Theorie) |
|---|---|---|---|---|---|
| 158 | 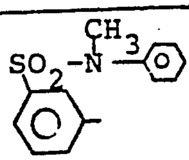 | 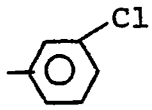 | 163°C | Chloroform | 68 |
| 159 | 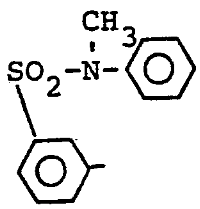 | 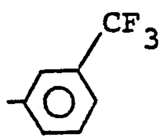 | 202°C | Chloroform | 62 |
| 160 | 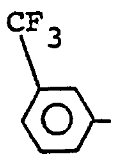 | 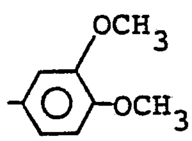 | 131°C | Chloroform | 81 |
| 161 | 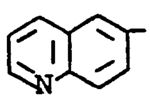 | 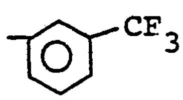 | 191°C | Chloroform | 76 |
| 162 | 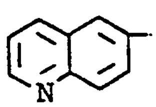 | 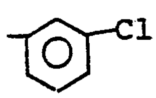 | 290°C | Chloroform | 67 |
| 163 | 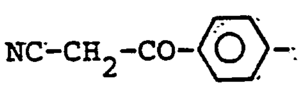 | 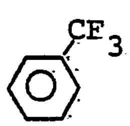 | 204°C | Essigester | 64 |
| 164 | 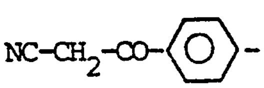 | 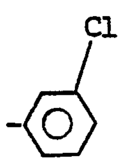 | 229°C | Essigester | 59 |

Patentansprüche

1. Verwendung von Harnstoffderivaten der allgemeinen Formel I

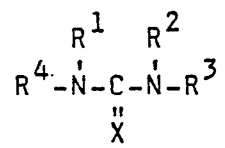 (I)

in welcher

$R^1$ und $R^2$ gleich oder verschieden sind und jeweils für Wasserstoff, geradkettiges, verzweigtes oder cyclisches Alkyl, für Aryl oder Aralkyl stehen, wobei die genannten Alkyl- und Arylreste ihrerseits gegebenenfalls substituiert sind durch Halogen oder Alkoxy,

$R^3$ und $R^4$ gleich oder verschieden sind und jeweils für einen Aryl- oder Heteroarylrest stehen, wobei diese Reste gegebenenfalls durch 1, 2, 3 oder 4 gleiche oder verschiedene Substituenten aus der Gruppe Nitro, Cyano, Halogen, Azido, Hydroxy, Amino, Carboxy, Aminocarbonyl, Aminosulfonyl substituiert sind, wobei die Aminogruppen jeweils durch Alkyl oder Aryl 1- oder 2-fach substituiert sein können, Alkoxycarbonyl, Acyloxy, Acylamino, SO-Alkyl, $SO_2$-Alkyl, Acyl, Phenyl, Phenoxy, Phenylmercapto, Alkyl, Alkoxy oder Alkylmercapto, wobei die genannten Alkyl-, Alkoxy- und Alkylmercaptoreste ihrereseits gegebenenfalls durch 1 oder mehrere Fluoratome substituiert sind und wobei die genannten Phenyl-, Phenylmercapto- und Phenoxysubstituenten ihrerseits wiederum substituiert sein können durch Halogen, Alkyl, Alkoxy oder Alkylmercapto, wobei die Alkyl-, Alkoxy- und Alkylmercaptoreste gegebenenfalls 1 oder mehrfach durch Fluor substituiert sind, oder wobei zwei benachbarte Substituenten am Arylrest gemeinsam mit den beiden Kohlenstoffatomen, an denen sie stehen, für einen gegebenenfalls durch Fluor substituierten Dioxan oder Dioxolring stehen.

X für Sauerstoff, Schwefel oder eine Cyanamidgruppe steht,

bei der Behandlung von Erkrankungen des Fettstoffwechsels.

2. Verwendung gemäß Anspruch 1 von Verbindungen der allgemeinen Formel I, in welcher

$R^1$ und $R^2$ gleich oder verschieden sind und jeweils für Wasserstoff, geradkettiges oder verzweigtes Alkyl mit 1 bis 4 Kohlenstoffatomen, Phenyl oder Benzyl stehen, wobei die genannten Alkyl-und Phenylreste ihrerseits gegebenenfalls substituiert sind durch Fluor oder Chlor,

$R^3$ und $R^4$ gleich oder verschieden sind und jeweils für einen Phenyl- oder Naphthylrest stehen, wobei diese Reste, insbesondere der Phenylrest, gegebenenfalls substituiert ist durch 1, 2, 3 oder 4 gleiche oder verschiedene Substituenten aus der Gruppe Nitro, Cyano, Halogen, insbesondere Fluor oder Chlor, Azido, Hydroxy, Amino, Carboxy, Aminocarbonyl, Aminosulfonyl, wobei die Aminogruppen jeweils durch Alkyl mit 1 bis 8 Kohlenstoffatomen oder Phenyl substituiert sein können, Phenyl, Phenoxy, Alkoxycarbonyl mit 1 bis 8 Kohlenstoffatomen, Acyloxy mit 1 bis 8 Kohlenstoffatomen, Acylamido mit 1 bis 8 Kohlenstoffatomen, Acyl mit 1 bis 8 Kohlenstoffatomen, $SO_2$-Alkyl mit 1 bis 8 Kohlenstoffatomen, oder durch Alkyl, Alkoxy oder Alkylmercapto jeweils 1 bis 8 Kohlenstoffatomen, wobei diese Alkyl-, Alkoxy- und Alkylmercapto-Reste ihrerseits gegebenenfalls durch Fluor ein- oder mehrfach substituiert sind und

X für Sauerstoff, Schwefel oder die Cyanamidgruppe steht.

3. Verwendung gemäß Anspruch 1 von Verbindungen der allgemeinen Formel I, in welcher

$R^1$ und $R^2$ jeweils für Wasserstoff stehen,
$R^3$ und $R^4$ gleich oder verschieden sind und jeweils für Phenyl stehen, das durch Halogen, insbesondere Fluor oder Chlor, Trifluormethyl, Trifluormethoxy, Trifluormethylmercapto, Cyano, Carboxy, Alkyl, Alkoxy, Acyl, Alkoxycarbonyl oder Dialkylaminosulfonyl, mit jeweils 1 bis 4 Kohlenstoffatomen in den Alkyl-, Alkoxy- und Acylresten, ein-, zwei-, drei- oder vierfach substituiert ist.

4. Verwendung gemäß Anspruch 1 von Verbindungen der allgemeinen Formel I, in welcher

$R^1$ und $R^2$ für Wasserstoff stehen,
X für Sauerstoff steht und
$R^3$ und $R^4$ jeweils für Phenyl stehen, welches durch 1, 2 oder 3 gleiche oder verschiedene Substituenten aus der Gruppe Chlor, Fluor, Cyano, Trifluormethyl, Trifluormethoxy, Trifluormethylmercapto, Dialkylaminosulphonyl, Alkoxycarbonyl oder Alkyl, mit jeweils 1 bis 4 Kohlenstoffatomen in den Alkyl- und Alkoxyresten, substituiert ist.

5. Lipidabsorptionshemmende Arzneimittel enthaltend mindestens eine Verbindung der allgemeinen Formel I gemäß Anspruch 1.

6. Verfahren zur Herstellung von lipidabsorptionshemmenden Arzneimitteln, dadurch gekennzeichnet, daß man mindestens eine Verbindung der allgemeinen Formel I gemäß Anspruch 1 mit üblichen Hilfs- und Trägerstoffen in eine geeignete Applikationsform überführt.

7. Verfahren zur Behandlung von Erkrankungen des Fettstoffwechsels dadurch gekennzeichnet, daß man Verbindungen gemäß der allgemeinen Formel I in Anspruch 1 Menschen oder Tieren im Bedarfsfalle appliziert.

8. N-(3-Chlor-4-trifluormethyl-phenyl)-N'-(3-trifluormethylphenyl)-harnstoff

0022958

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 80 10 3765

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CH - A - 322 815 (WANDER)<br>* Ansprüche *<br>-- | 1-6 |
| X | DE - C - 924 864 (HOECHST)<br>* Ansprüche *<br>-- | 1-6 |
| X | DE - C - 962 699 (SCHERING)<br>* Ansprüche *<br>-- | 1-6 |
| X | DE - B - 1 221 638 (HOECHST)<br>* Ansprüche *<br>-- | 1-6 |
| X | GB - A - 969 808 (BOOTS PURE DRUG CY)<br>* Ansprüche *<br>-- | 1-6 |
| X | GB - A - 1 059 034 (ICI)<br>./. | 1-6 |

KLASSIFIKATION DER ANMELDUNG (Int Cl 3)

A 61 K 31/17
C 07 C 127/19
157/09
149/437
147/00
143/58
143/72
C 07 D 521/00//
(C 07 D 521/00
317/46
279/02
251/22
295/14
215/42
./.

RECHERCHIERTE SACHGEBIETE (Int Cl 3)

C 07 C 127/19
157/09

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6,8

Unvollständig recherchierte Patentansprüche:

Nicht recherchierte Patentansprüche: 7

Grund für die Beschränkung der Recherche: Verfahren zur chirurgischen oder therapeutischen Behandlung des menschlichen oder tierischen Körpers (Siehe Art. 52(4) des Europäischen Patentübereinkommens).

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 30-10-1980 | GAUTIER |

Europäisches Patentamt

**EUROPÄISCHER TEILRECHERCHENBERICHT**

Nummer der Anmeldung
EP 80 10 3705
-2-

| EINSCHLÄGIGE DOKUMENTE | | | KLASSIFIKATION DER ANMELDUNG (Int Cl.3) |
|---|---|---|---|
| Kategorie | Kennzeichnung des Dokuments mit Angabe, soweit erforderlich, der maßgeblichen Teile | betrifft Anspruch | C 07 D 249/06<br>213/75<br>239/42<br>215/38<br>277/48<br>279/08<br>307/91<br>277/82) |
| | * Ansprüche * | | |
| | -- | | |
| X | FR - M - 3 361 (CIBA)<br>* Ansprüche * | 1-6 | |
| | -- | | |
| X | FR - M - 2 104 (MADAN)<br>* Ansprüche * | 1-6 | |
| | -- | | RECHERCHIERTE SACHGEBIETE (Int. Cl.3) |
| X | FR - A - 2 220 255 (PFIZER)<br>* Ansprüche; Seiten 11,33 * | 1-6 | |
| | -- | | |
| X | DE - C - 859 151 (CHEMIE GRUNENTHAL)<br>* Ansprüche * | 1-6 | |
| | -- | | |
| X | FR - M - 3 969 (ICI)<br>* Ansprüche * | 1-6 | |
| | -- | | |
| X | FR - A - 1 063 842 (CIBA)<br>* Ansprüche * | 1-6 | |
| | -- | | |
| X | FR - A - 1 067 900 (CIBA)<br>* Ansprüche * | 1-6 | |
| | -- | | |
| X | FR - A - 1 073 588 (CIBA)<br>* Ansprüche * | 1-6 | |
| | -- ./. | | |

Europäisches Patentamt

# EUROPÄISCHER TEILRECHERCHENBERICHT

Nummer der Anmeldung EP 80 10 3765

-3-

| EINSCHLÄGIGE DOKUMENTE | | | KLASSIFIKATION DER ANMELDUNG (Int Cl 3) |
|---|---|---|---|
| Kategorie | Kennzeichnung des Dokuments mit Angabe, soweit erforderlich, der maßgeblichen Teile | betrifft Anspruch | |
| X | FR - A - 1 078 457 (CIBA)<br>* Ansprüche *<br>-- | 1-6 | |
| X | FR - A - 1 080 728 (CIBA)<br>* Ansprüche *<br>-- | 1-6 | |
| X | US - A - 2 688 039 (CIBA)<br>* Ansprüche *<br>-- | 1-6 | RECHERCHIERTE SACHGEBIETE (Int Cl 3) |
| | FR - A - 1 109 430 (GEIGY)<br>* Ansprüche; Beispiele *<br>---- | 8 | |